# EUROPEAN PATENT SPECIFICATION

(11) **EP 2 219 478 B1**
(45) Date of publication and mention of the grant of the patent: **10.05.2017**
(21) Application number: 08861480.5
(22) Date of filing: 17.12.2008
(51) Int. Cl.: A23L 15/00

(54) **METHOD OF PRODUCING EGG YOLK BASED FUNCTIONAL FOOD PRODUCT AND PRODUCTS OBTAINABLE THEREBY**
VERFAHREN ZUR HERSTELLUNG EINES FUNKTIONELLEN NAHRUNGSMITTELPRODUKTS AUF EIGELBBASIS UND AUF DIESE WEISE HERSTELLBARE PRODUKTE
PROCÉDÉ DE PRODUCTION DE PRODUITS ALIMENTAIRES FONCTIONNELS À BASE DE JAUNE D' UF ET PRODUITS POUVANT ÊTRE OBTENUS À L'AIDE DE CELUI-CI

(30) Priority: 17.12.2007 EP 07123322
(43) Date of publication of application: 25.08.2010
(73) Proprietor: Newtricious B.V., 5808 AL Oirlo (NL)
(72) Inventor: THIELEN, Wilhelmus Johannes Gertrudes, NL-5803 AV Venray (NL); BERENDSCHOT, Toussaint Theresia Johannes Maria, NL-6212 BG Maastricht (NL); NELISSEN, Joseph Wilhelmus Petrus Maria, NL-5808 AL Oirlo (NL); PLAT, Jogchum, NL-6243 CP Geulle (NL)
(74) Representative: Habets, Winand
(86) International application number: PCT/NL2008/050810
(87) International publication number: WO 2009/078716

(56) References cited:
- EP-A- 1 068 806
- EP-A- 1 155 627
- WO-A-00/38535
- US-A- 5 415 879
- US-A- 6 149 964
- US-A1- 2005 053 713
- ANNU. REV. NUTR., [Online] 2006, pages 75-103, XP002485599 Retrieved from the Internet: URL:http://arjournals.annualreviews.org/do i/pdf/10.1146/annurev.nutr.25.050304.09260 5?cookieSet=1> [retrieved on 2008-06-24]

## Description

### Field of the invention

The present disclosure relates to the field of food products, especially food products specifically designed for providing health benefits beyond the basic function of nutrition for a subject consuming the product on a regular basis, e.g. on a daily basis. In particular, the disclosure concerns a method of producing a food product containing significant amounts of certain pharmacologically active nutrients, which food product provides an excellent matrix for absorption of said nutrients in the gastrointestinal tract. The food products as disclosed herein are furthermore formulated such as to render them suitable for use on a regular basis for a significant period of time, e.g. months or even years. The present disclosure thus provides methods of producing these food products, which may be referred to as functional foods or nutraceuticals, as well as the functional food products obtainable by said methods. Also disclosed herein are methods of supplementing the nutrients in a subject in need thereof, said method involving the consumption by said subjects of the food products as described herein.

### Background of the invention

There is an increasing trend in society to consider food from a functional viewpoint. Functional foods, or nutraceuticals, can be defined as those food products that provide health benefits beyond the basic function of foods as nutrition. Interest in functional foods increased in the last decade due to the overwhelming scientific evidence highlighting the relation between diet and health. The interest in this area has resulted in a number of new foods in the marketplace designed to address specific health concerns, particularly with regard to chronic diseases related to aging as well as conditions related to the so-called modem western diet. About 1.5% of the total food market can be classified as the functional food market. Growth rates of over 10% in recent years show the dynamics of this special market.

Some functional food ingredients that are of interest in this area include omega-3 fatty acids, especially DHA and EPA; vitamin D; Vitamin E; Folic acid; xanthophylls; iodine and selenium.

Omega-3 fatty acids from fish oils are claimed among others to have an anti-inflammatory effect in various autoimmune diseases and to reduce the risk for cardiovascular disease and stroke. In Japan these products are marketed for performance enhancement. In addition to bread, eggs and breakfast cereals, functional food drinks containing omega-3 fatty acids are also on the market. Docosahexaenoic acid (DHA) is an omega-3 fatty acid. Dietary DHA can reduce the level of blood triglycerides in humans, which may reduce the risk of heart disease. Low levels of DHA result in reduction of brain serotonin levels and have been associated with ADHD, Alzheimer's disease and depression and there is mounting evidence that DHA supplementation may be effective in treating or preventing such diseases or, at least, some of the symptoms thereof.

Vitamin D is a group of fat-soluble prohormones and plays an important role in the maintenance of organ systems. Vitamin D deficiencies result in impaired bone mineralization and lead to bone softening diseases and possibly contribute to osteoporosis. Very few foods are naturally rich in vitamin D, and most vitamin D intake nowadays is in the form of fortified milk, soy milk and cereal grains. Natural foods that do contain some Vitamin D include fish liver oils, fatty fish species, mushrooms and egg.

Vitamin E or Tocopherol is used to denote a series of methylated phenols, which are fat-soluble antioxidants. Alpha-tocopherol is traditionally recognized as the most important biological antioxidant. In the diet the most important sources of vitamin E include vegetable oils, nuts, sunflower seeds, certain fruits and vegetables and wheat germ. Although in some cases the evidence is still controversial research has lead to a widely held belief that sufficient intake of vitamin E may help or prevent coronary heart disease; may help to protect against the development of cancers; may help to delay or prevent cataract growth; may have a protective effect against age-related macular degeneration; may help protect the retina from glaucomatous damage; may help reduce the onset of Alzheimer's disease; may help protect against Parkinson's disease and might help to inhibit the neural death caused in the hippocampus by THC.

Folic acid is a form of the water soluble vitamin B9. Leaf vegetables such as spinach and turnip, dried beans and peas, sunflower seeds, certain fruits and fortified cereal products are amongst the most important dietary sources of folic acid. Most interest in dietary folic acid intake concerns its protective effect against a number of congenital malformations during pregnancy. Though still controversial, some evidence has associated low blood levels of folate with a greater risk of cancer, a greater risk of heart disease and stroke, depression and decreases in short-term memory, mental agility and verbal fluency.

The xanthophylls, especially zeaxanthin and lutein, are naturally occurring carotenoids. They are mainly found in the human diet in fruits and vegetables, especially green vegetables such as spinach, and eggs. There is epidemiological evidence of a relationship between low plasma concentrations of lutein and zeaxanthin on the one hand and age-related macular degeneration (AMD) on the other. It is nowadays common belief that lutein and/or zeaxanthin supplementation indeed protect against AMD. Improved skin health is also a benefit of dietary consumption of xanthophylls. Furthermore it has been suggested that there may be a correlation between low plasma levels of xanthophylls and incidence of cataract, atherosclerosis and breast and colon cancer.

Iodine is an essential trace element. The US FDA recommends iodine intake through the diet for both men and women for proper production of thyroid hormone. Iodine deficiency gives rise to hypothyroidism, symptoms of which are extreme fatigue, goitre, mental slowing, depression, weight loss and low basal body temperature. Iodine deficiency is also the leading cause of preventable mental retardation, an effect which happens primarily when babies and small children become hypothyroid by lack of the element. The natural sources of iodine include certain sea foods as well as vegetables grown on iodine rich soil. The addition of iodine to table salt has largely eliminated iodine-deficiency associated problems in western nations.

Selenium is also an essential micronutrient for animals. In humans selenium is a trace element nutrient which functions as cofactor for reduction of antioxidant enzymes such as glutathione peroxidases and thioredoxin reductase. It also plays a role in the functioning of the thyroid gland by participating as a co-factor for thyroid hormone deiodinases. Dietary selenium comes from nuts, cereals, meat, fish and eggs.

US2005053713 A1 discloses a commercial baby food composition comprising dried egg yolks and non-fat dried milk. It also discloses methods for enhancing cognitive ability in infants as well as methods of making DHA-enriched baby food compositions and providing those to a consumer.

US 5773072 A discloses a process for preparing a heat-stable oil and water emulsion sauce which comprises homogenizing a mixture of unmodified egg yolk and of diacetyl tartaric acid ester of monoglyceride ("DATEM") emulsifying agents to obtain a homogenized mixture and combining the homogenized mixture with ingredients comprising an edible oil, water, a thickener component and an ingredient selected from the group consisting of salt and sugar to obtain a further mixture and so that the further mixture comprises, by weight, the oil in an amount of from 5% to 70%, the unmodified egg yolk in an amount of from 0.1% to 20% and the DATEM in an amount of between 0.5% and 1.5% (dry weight) and homogenizing the further mixture to obtain an emulsion which is heat-stable, and then heating the emulsion at a temperature and for a time to at least pasteurize the emulsion to obtain a heat-treated emulsion product.

Many studies have shown that a large number of individuals do not manage to take in adequate amounts of these nutrients, in general, because the natural sources providing them have increasingly been eliminated from the normal diet. While all these nutrients can be obtained in tablet or capsular forms it is generally held that their supply in a more appealing food product form is a valuable option in supplementing these nutrients.

As will be understood from the above, there is a public as well as a commercial interest in the development of functional foods that can provide a suitable dietary source of one or more of these and/or other pharmacologically active nutrients.

It is therefore an objective of the present disclosure to provide suitable functional food products containing these and/or other pharmacologically active nutrients.

In particular, it is an objective of the present disclosure to provide such functional food products which are formulated such as to provide a matrix from which these pharmacologically active nutrients are absorbed efficiently in the gastrointestinal tract, such as to assure adequate bio-availability thereof.

It is also in particular an objective of the present disclosure to provide such functional food products that are formulated with a view to achieving high compliance by the subject taking the functional food product. This typically means that the consumer is willing to take the product as long as needed and as often as is needed to provide the recommended amounts of the nutrients, typically once daily for months, or even years, without growing disinterest, dislike or even aversion towards the product, resulting in non-compliance. Factors of interest in that regard typically include appearance, palatability, texture, taste and flavour, ease of use and the like.

### Summary of the invention

According to the present inventors it has proven feasible, i.e. also on an industrial and in a commercially attractive way, to produce eggs, in particular chicken eggs, that are enriched in one or more pharmacologically active nutrients, such as those selected from omega-3 fatty acids, especially DHA; vitamin D; Vitamin E; Folic acid; xanthophylls; iodine and selenium. It was found that significant fractions of such pharmacologically active nutrients were efficiently deposited into the yolks of the eggs produced by poultry fed with a fodder enriched in these nutrients.

According to the prior art, this concept as such had been explored and described before, notably in US patent application no. 2006/0171995 and European patent application no. 1 591 018

US 2006/0171995 describes the absorption and deposition of carotenoids in the eggs of poultry fed with a carotenoid enriched diet. It is described that eggs could be produced in this way containing in excess of 1.0 mg carotenoids per gram of edible portion of the egg.

EP 1 591 018 concerns a composition for feeding layer hens containing micronutrients in amounts sufficient to obtain eggs rich in said micronutrients. EP 1 591 018 suggests the use of compositions containing selenium, iodine, vitamin E, vitamin D3, vitamin B1, vitamin B6, folic acid and/or carotenoids, e.g. zeaxanthine as well as alpha-linolenic acid and/or docosahexaenoic acid and describes the amounts of each of these micronutrients in the eggs that can be produced accordingly.

The actual use of these eggs for supplementing the individual's diet has not in fact been disclosed in these prior art documents. Moreover, although it is suggested that such eggs are able to deliver the specific nutrients to an individual consuming the egg, it is not clear whether it would actually be feasible to use these eggs as a main dietary source of said nutrients in view of the maximum amount of the nutrient that can be deposited in the egg yolk (taking into account regulatory constraints on the feeding compositions), the bio-availability of the nutrients from the eggs and the amount of eggs that would accordingly be needed in the diet. The prior art documents do not mention or suggest any other food products besides the eggs.

It has now been found by the inventors that egg yolk obtained from the aforementioned eggs can suitably be used in high amounts as a component of a functional food product fulfilling all the requirements mentioned herein before.

More in particular, clinical studies, which will be discussed in more detail hereafter, have shown that functional food products as disclosed herein are suitable for providing significant amounts of the pharmacologically active nutrients with sufficient bio-availability. Moreover, it was unexpectedly found that the composition obtained by combining the egg yolk and aqueous dispersion as disclosed herein provides an excellent carrier matrix from which said nutrients, including highly lipophilic ones, could be absorbed very efficiently. Without wishing to be bound by any particular theory, it is hypothesized that this is mainly due to the presence of the egg yolk phospholipids acting as emulsifiers and/or stabilizers. The functional food products as disclosed herein do not require the incorporation of further emulsifying agents to assure sufficient bio-availability of lipophilic pharmacologically active nutrients.

It was furthermore found that sufficient amounts of the egg yolk could be included in volumes of functional food products, small enough to be formulated as daily "shots", as is generally desired for this type of product.

It was also found that these high relative amounts of egg yolk did not adversely affect appearance, texture, taste and/or flavour of products as disclosed herein such as those formulated as yoghurts, fruit drinks, milk drinks or puddings.

It was also shown in these studies that the functional food products essentially containing a combination of egg yolk and an aqueous dispersion as disclosed herein were well accepted by the test subjects during the entire study period of three months.

The functional food products as disclosed herein can be formulated to have shelf stability which is generally considered sufficient for this type of products, both in terms of microbial spoilage and emulsion or suspension stability.

Finally, it was found that functional food products as disclosed herein can be formulated that upon regular, e.g. daily, use did not give rise to significant increases in plasma cholesterol levels contrary to what is observed in individuals supplementing their diets with corresponding amounts of egg.

These and other benefits of the present disclosure will become more apparent to the skilled person from the detailed description and the examples here below.

### Detailed description of the invention

In accordance with a first aspect, the present disclosure thus provides a method of producing a functional food product comprising mixing, in a ratio within the range of 2-7:
i) a combination of water and one or more other food grade materials, preferably in the form of an aqueous dispersion or solution thereof, with
ii) egg yolk, wherein the egg yolk is obtained by feeding poultry a diet enriched in at least one pharmacologically active nutrient, collecting the eggs produced by said poultry and taking therefrom the yolk.

As used herein the expression "functional food product", refers to a class of processed foods that claims to promote good or improved health, to prevent disease, or the like, beyond the basic function of food which is to supply nutrients to the body. By including a sufficient amount of the egg yolk obtained in accordance with the invention in a food product, this is typically achieved.

The present functional food product typically is a so-called ready-to-use product, which means that the product does not require any further processing, such as cooking or baking or mixing with other ingredients, before it is suitable for consumption.

The functional food product as disclosed herein is a product selected from the group of beverages, such as fruit flavoured drinks or dairy type drinks; and desserts, such as puddings, custards or spoonable yoghurts.

It is furthermore particularly preferred that the functional food product is provided in unit dosage form. A unit dosage form, in accordance with its regular meaning, refers to a form wherein predetermined portions of the product are provided in discrete packages, said portions corresponding to the amount to be taken per administration event. A particularly preferred example includes discrete packages containing a portion corresponding to the daily dosage. More in particular discrete packages are preferably provided containing a single portion to be consumed as a single "shot", "snack", "refreshment" or "bite".

In accordance with the above, the present disclosure provides closed or sealed packages containing a functional food product as defined herein before in an amount of 50-150 ml, preferably 75 - 125 ml.

However, the disclosure also provides a package containing a multitude of such dosages or portions of the functional food product, i.e. which are not in discrete unitary dosage packages. Typically such packages contain the product in an amount of 250-1000 ml, preferably 500 - 1000 ml.

The term "comprising", as used herein, is meant not to be limiting to-any subsequently stated elements but rather to encompass non-specified elements of major or minor functional importance. In other words the listed steps, elements or options need not be exhaustive. Whenever the words "including", "containing" or "having" are used, these terms are meant to be equivalent to "comprising" as defined above.

The term "egg yolk", as used herein may refer simply to untreated egg yolk as separated from the egg white after a shell egg is broken, but also to heat sterilized egg yolk, frozen egg yolk that has been thawed, rehydrated egg yolk that is obtained by adding water to dried powdered egg yolk to the extent of normal egg yolk, and egg yolk subjected to various treatments such as lyso-conversion, decholesterolization, lyso-decholesterolization, etc. The "term egg-yolk solids" is intended to mean the solids present in natural egg yolk or in dried egg-yolk products such as those commonly used as ingredients in the food industry. The amounts of solids in egg yolk from chickens eggs and dried egg-yolk products, can be determined using known methods, and are typically about 46% and about 96%, respectively.

In the art Poultry is the category of domesticated birds kept for meat and eggs. These most typically are members of the order Galliformes (which includes chickens and turkeys). Preferably, the term poultry is used to refer to chickens. The term "pharmacologically active nutrient" refers to any substance that can be found in food products forming part of a normal healthy diet, usually in low or trace amounts, and having a pharmacological effect, meaning that it has therapeutic or prophylactic effectiveness. The term pharmacologically active nutrient thus does not encompass the bulk food ingredients which function as the energy sources and/or building blocks in the normal metabolic processes of the human or animal body. Suitable examples of such pharmacologically active nutrients include omega-3 fatty acids, especially DHA (docosahexaenoic acid) and EPA (eicosapentaenoic acid), vitamin D, Folic acid, Vitamin E, xanthophylls, iodine, selenium and zinc. It is therein preferred that the pharmacologically active ingredient is selected from EPA, vitamin D, Folic acid, Vitamin E, xanthophylls, iodine and selenium, more preferably from vitamin D, Folic acid, Vitamin E, xanthophylls, iodine and selenium. It is therein particularly preferred that the pharmacologically active nutrient is a lipophilic pharmacologically active nutrient for above-stated reasons.

As mentioned before, the egg yolk is obtained by feeding poultry a diet enriched in at least one pharmacologically active nutrient and collecting the eggs produced by said poultry and taking therefrom the yolk. As used herein "enriched" has the meaning of containing additional amounts of said pharmacologically active nutrient as compared to standard poultry diets. Typically the diet as disclosed herein is enriched with said pharmacologically active nutrient to the extend that it contains 125 %, preferably 150 %, more preferably 200 % and most preferably 300 % of the amount of said nutrient normally consumed, e.g. on a daily basis, by said poultry.

In accordance with the present disclosure, the poultry is fed a diet enriched in at least two, preferably at least three, more preferably at least four and most preferably at least five pharmacologically active nutrients.

Furthermore, in accordance with the present disclosure, the diet fed to the poultry comprises omega-3 fatty acids in an amount of at least 5 g/kg of the diet, more preferably within the range of 10-50 g/kg; and/or vitamin D in an amount of at least 1500 IU, more preferably within the range of 2000-5000 IU; and/or Folic acid in an amount of at least 10 mg/kg, more preferably within the range of 20-100 mg/kg; and/or Vitamin E in an amount of at least 20 mg/kg, more preferably within the range of 100-500 mg/kg; and/or xantophylls in an amount of at least 10 mg/kg, more preferably within the range of 20-100 ppm; and/or Iodine in an amount of at least 0.75 mg/kg, more preferably within the range of 1-5 mg/kg; and/or Selenium in an amount of at least 0.2 mg/kg, more preferably within the range of 0.2-0.3 mg/kg and/or zinc in an amount of at least 30 mg/kg, preferably in an amount of at least 50 mg/kg.

The expression "IU" as used herein stands for International Units, which is commonly used in the art for expressing the quantity of e.g. a vitamin in terms of biological activity.

Typically feeding the poultry diets enriched in one or more nutrients as described here before will result in the poultry producing eggs containing omega-3 fatty acids in an amount of at least 100 mg/ 100 g, preferably at least 150 mg/ 100 g; and/or vitamin D in an amount of at least 1 µg/ 100 g, preferably at least 1.5 µg/ 100 g; and/or Folic acid in an amount of at least 150 µg/ 100 g, preferably at least 250 µg/ 100 g; and/or Vitamin E in an amount of at least 2 mg/ 100 g, preferably at least 3 mg/ 100 g; and/or xantophylls in an amount of at least 500 µg/ 100 g, preferably at least 700 µg/ 100 g; and/or Iodine in an amount of at least 60 µg/ 100 g, preferably at least 90 µg/ 100g; and/or Selenium in an amount of at least 10 µg/ 100 g, preferably at least 15 µg/ 100g and/or zinc in an amount of at least 2.5 mg/ 100 g, preferably at least 3 mg/ 100g.

As stated before, these eggs are collected and the yolk is taken therefrom. Typically the egg yolk is separated from the egg white using an automated process, although this is not an essential characteristic of the disclosure. A non-limiting example of an apparatus for performing this kind of processing has been described in EP1205140.

In accordance with the present disclosure, a method is provided as described above, wherein the combination of water and the at least one food-grade material on the one hand and the egg yolk on the other hand are mixed in a ratio within the range of 3-6, most preferably within the range of 3.5-5.

The food grade material as disclosed herein typically comprises at least one material selected from the group of carbohydrates, proteins and lipids. Suitable examples of carbohydrates, proteins and lipids are as listed herein below.

In accordance with the present disclosure the combination of water and the at least one food grade material are in the form of an aqueous dispersion or solution prior to mixing with the egg yolk. Typically said dispersion or solution at least comprises a dissolved or dispersed carbohydrate, protein or lipid material. Typically said food-grade material is comprised in said aqueous dispersion or solution in an amount of at least 1 wt%, preferably at least 2 wt%, most preferably at least 5 wt%. Typically said amount may be as high as 80 wt%. Preferably it does not exceed 50 wt%, most preferably it does not exceed 25 wt%.

In accordance with the present disclosure, a method is provided as defined herein before, wherein egg yolk is combined with an aqueous dispersion selected from the group consisting of skimmed milk, semi-skimmed milk, buttermilk, fermented milk, especially yoghurt, soy drink or soy milk, fermented soy milk, fruit juices, fruit purees, syrups, vegetable juices, vegetable purees and combinations thereof.

It was surprisingly found that the daily use of a product as disclosed herein wherein egg yolk and butter milk have been combined does not give rise to significant alterations in plasma cholesterol levels, whereas the (daily) intake of a corresponding amount of egg enriched in an pharmacologically active ingredient did give rise to significant increases in plasma cholesterol. These findings are described in more detail in the examples. Without wishing to be bound by any theory it is hypothesized that the absence of plasma cholesterol increasing effects may be ascribable to the polar lipids, especially sphingolipids, naturally found in dairy products such as butter milk.

Therefore, as disclosed herein, a method is provided as defined herein before, wherein the combination of water and one or more other food grade materials comprises buttermilk, skimmed milk, semi-skimmed milk, fermented milk, especially yoghurt, fractions thereof and mixtures thereof. Most preferably it comprises whole buttermilk or a buttermilk fraction.

Also disclosed herein is a method as defined herein before, wherein the combination of water and one or more other food grade materials comprises polar dairy lipids, especially polar dairy lipids selected from the group of sphingolipids and phospholipids, preferably from the group of sphingolipids, e.g. ceramide and/or sphingomyelin. Preferably the combination of water and at least one further food grade material comprises, based on the total weight of the functional food product, at least 0.05 wt%, more preferably at least 0.08 wt% of said polar dairy lipids.

It is also hypothesized that the bacteria present in yoghurt and buttermilk may cause or contribute to the absence of adverse effects on plasma lipids and cholesterol observed. Therefore also disclosed herein is a product as defined herein before comprising an effective amount, e.g. 10⁴ cfu/ml or more, preferably 10⁵ cfu/ml or more, most preferably 10⁶ cfu/ml or more, of lactic acid bacteria, more preferably bacteria selected from Lactobacillus and Lactococccus, more preferably from *L. Lactis, L. Acidophilus, L. Casei, L. Reuteri, L. Gasseri* and *L. Delbrueckii,* most preferably from *L. Acidophilus, L. Delbrueckii* and *L. Casei.*

Optionally further ingredients may be added as desired. Hence, also disclosed herein is a method as defined herein before, comprising the step of adding one or more additional ingredients selected from the group consisting of colouring agents, flavourings, sweeteners, preservatives, anti-oxidants, viscosity modifying agents, emulsifiers, food acids and combinations thereof. Preferably the method as disclosed herein additionally comprises the steps of mixing, e.g. using shear, and/or pasteurizing or sterilizing the mixture that is obtained in accordance with the above described method.

Preferably, the method does not comprise a step wherein ethanol is added to the product or wherein the product is processed such as to produce a liquor or alcoholic beverage.

The present disclosure provides a method as defined herein before, said method further comprising filling a package, preferably a package as described herein before, with the functional food product and sterilizing or pasteurizing said package before or after closing or sealing said package.

A second aspect of the disclosure concerns the functional food product that is obtainable by the method as defined herein before.

The functional food product of the disclosure comprises a mixture of the egg yolk and a combination of water and at least one further food-grade material, as defined herein before.

Also disclosed herein is a food product wherein said combination of water and at least one further food-grade material is a dispersion selected from the group of skimmed milk, semi-skimmed milk, buttermilk, fermented milk, especially yoghurt, soy drink or soy milk, fermented soy milk, fruit juices, fruit purees, syrups, vegetable juices, vegetable purees and combinations thereof. As explained before, the present disclosure preferably concerns a product comprising buttermilk, skimmed milk, semi-skimmed milk, fermented milk, especially yoghurt, fractions thereof and mixtures thereof. The present disclosure preferably concerns a product as defined before comprising one or more polar dairy lipids, especially polar dairy lipids selected from the group of sphingolipids and phospholipids, preferably from the group of sphingolipids. Said lipids are preferably present in a total amount of at least 0.05 wt%, more preferably at least 0.08 wt%. The present disclosure also preferablyconcerns a product as defined before comprising an effective amount, e.g. 10⁴ cfu/ml or more, preferably 10⁵ cfu/ml or more, most preferably 10⁶ cfu/ml or more, of lactic acid bacteria, more preferably of bacteria selected from Lactobacillus and Lactococccus, more preferably from *L. Lactis, L. Acidophilus, L. Casei, L. Reuteri, L. Gasseri* and *L. Delbrueckii,* most preferably from *L. Acidophilus*, *L. Delbrueckii* and *L. Casei.*

The present disclosure also provides a functional food product comprising a combination of the egg yolk, water at least one further food-grade material selected from the group of carbohydrates, proteins and lipids, and optionally one or more additional ingredients such as colouring agents, flavourings, preservatives, anti-oxidants, viscosity modifying agents, e.g. starch or gelatine, emulsifiers, food acids and the like. It is noted that, although the functional food products of the disclosure do not require the addition of any emulsifying agent, due to the presence of the egg yolk phospholipids, products containing additional emulsifying agents, for whatever purpose, are encompassed by the present disclosure.

The disclosure also provides a functional food product as defined herein before, wherein the amount of egg yolk solids is within the range of 0.025-0.4 g/ml of the functional food product. Also provided by the present disclosure is a functional food product wherein the amount of egg yolk solids is within the range of 0.035-0.3 g/ml, more preferably, most preferably 0.05-0.2 g/ml. As will become apparent from the description hereafter this roughly corresponds to the product containing the complete yolk of 1 chicken egg per 50-150 ml, preferably 75-125 ml. In this document, the amount of egg yolk is expressed as dry solids weight per millilitre of the functional food product. As will be understood by the skilled person, this is not intended to imply that egg yolk is necessarily added in dry solid form, although such is encompassed by the present disclosure.

Furthermore, , a functional food product as defined herein before is provided, containing omega-3 fatty acids in an amount of at least 50 mg/ 100 ml of the functional food product, preferably at least 75 mg/ 100 ml of the functional food product; and/or vitamin D in an amount of at least 0.5 µg/ 100 ml of the functional food product, preferably at least 0.75 µg/ 100 ml of the functional food product; and/or Folic acid in an amount of at least 75 µg/ 100 ml of the functional food product, preferably at least 125 µg/ 100 ml of the functional food product; and/or Vitamin E in an amount of at least 1 mg/ 100 ml of the functional food product, preferably 1.5 mg/ 100 ml of the functional food product; and/or xantophylls in an amount of at least 250 µg/ 100 ml of the functional food product, preferably at least 350 µg/ 100g egg yolk; and/or Iodine in an amount of at least 30 µg/ 100 ml of the functional food product, preferably at least 45 µg/ 100 ml of the functional food product; and/or Selenium in an amount of at least 5 µg/ 100 ml of the functional food product, preferably at least 7.5 µg/ 100 ml of the functional food product; and/or zinc in an amount of at least 1.5 mg/ 100 ml, preferably 2.5 mg/ 100 ml.

As disclosed herein, said functional food product thus contains carbohydrates such as mono-, di- and/or trisaccharide and oligo and/or poly-saccharides. Preferably said functional food products contains one or more mono-, di- and/or trisaccharides selected from the group of glucose, fructose, maltose, sucrose, galactose and lactose, in an amount ranging from 0.5 -10 wt%, based on the total weight of the product, more preferably 1-7.5 wt%, most preferably 2-6 wt%. Also as disclosed herein, the functional food product contains one or more fibers, preferably selected from the group of ionic non-starch polysaccharides, including alginates, pectins (including amidated pectins), carrageenans, xanthans, gellans, furcellarans, karaya gum, rhamsan, welan, gum ghatti, gum arabic and salts; neutral non-starch polysaccharides, including galactamannan, guar gum, locust bean gum, tara gum, ispaghula (= psyllium), beta-glucans, konjac mannans, methylcellulose, gum tragacanth, detarium, tamarind, as well as fructo-oligosaccharides (FOS), galacto-oligosaccharides (GOS), inulin, celluloses, chemically-modified celluloses and vegetable and/or fruit fibres. Preferably the amount of fibers contained in the food product is within the range of 0.1-20 wt%, more preferably within the range of 0.2-10 wt%, most preferably within the range of 0.5-7.5 wt%.

Furthermore, as disclosed herein, the functional food product contains proteins and/or peptides other than the egg yolk protein, preferably selected from the group of dairy proteins; vegetable proteins; meat derived proteins and fish derived proteins as well as partial hydrolysates thereof, especially from the group consisting of whey protein, caseinates, soy protein, pea protein, maize protein, wheat protein, gelatin, albumin, their partial hydrolysates and combinations thereof, in an amount ranging from 0.1-20 wt%, based on the total weight of the product, more preferably 0.2-10 wt%, most preferably 0.5-7.5 wt%.

Preferably, the functional food product contains less than 5 wt% of egg white albumin, more preferably less than 3.5 wt%, still more preferably less than 2.0 wt%, and most preferably less than 1.0 wt%.

Furthermore, as disclosed herein, the functional food product contains lipid materials other than the egg yolk lipids, preferably selected from the group of milk fat, butter fat and vegetable oils, animal fats and fish oils preferably from the group of milk fat and butter fat, in an amount ranging from 0.05-7.5 wt%, based on the total weight of the product, more preferably 0.1-5 wt%, most preferably 1.5-3 wt%.

In addition, the functional food product of the disclosure may comprise additional ingredients such as colouring agents, flavourings, preservatives, anti-oxidants, viscosity modifying agents, emulsifiers, food acids and the like, which will be applied in conventional amounts known to the skilled person.

As further disclosed herein, the functional food product comprises little or no ethanol. Preferably the amount of ethanol in the functional food product is below 5 wt%, based on the total weight of the functional food product, more preferably it is below 1 wt%, still more preferably it is below 0.5 wt%. Most preferably the functional food product does not contain any ethanol.

Also disclosed herein is a functional food product as described herein before, for use as a medicament. As explained herein before, each of the pharmacologically active nutrients of the compositions as described herein has been implicated in a variety of conditions and disorders. It is also disclosed herein that the at least one pharmacologically active nutrient is omega-3 fatty acid and the functional food products is intended for reducing the incidence and/or severity of AMD, cardiovascular disease, stroke, ADHD, Alzheimer's disease or depression. It is also disclosed herein that the at least one pharmacologically active nutrient is vitamin D and the functional food products is intended for reducing the incidence and/or severity of bone softening diseases, osteoporosis or vitamin D deficiency. It is also disclosed herein that the at least one pharmacologically active nutrient is vitamin E and the functional food products is intended for reducing the incidence and/or severity of cardiovascular disease, cataract growth, age-related macular degeneration, glaucomatous damage of the retina, Alzheimer's disease, Parkinson's disease or vitamin E deficiency. It is also disclosed herein that the at least one pharmacologically active nutrient is folic acid and the functional food products is intended for reducing incidence and/or severity of cardiovascular disease, depression or decreases in short-term memory, mental agility and/or verbal fluency. It is also disclosed herein that the at least one pharmacologically active nutrient is a xanthophyll, especially lutein or zeaxanthine, and the functional food products is intended for reducing the incidence and/or severity of age-related macular degeneration, cataract, atherosclerosis and/or for improving skin health. It is also disclosed herein that the at least one pharmacologically active nutrient is Iodine and the functional food products intended for reducing the incidence and/or severity of hypothyroidism or iodine deficiency. Finally, It is also disclosed herein that the at least one pharmacologically active nutrient is selenium and the functional food products is intended for reducing the incidence and/or severity of selenium deficiency.

Yet another aspect of the invention concerns a method of treating or preventing any of the conditions or disorders described here above in a subject by administering to said subject a sufficient amount of the functional food product, wherein the at least one pharmacologically active nutrient as disclosed herein is the one implicated in said condition or disorder.

Another aspect of the disclosure concerns the use of egg yolk that is obtained by feeding poultry a diet enriched in at least one pharmacologically active nutrient, preferably a pharmacologically active nutrient selected from the group consisting of omega-3 fatty acids, especially DHA and EPA; vitamin D; Folic acid; Vitamin E; xantophylls, especially lutein and zeaxanthin; iodine; selenium; and zinc, collecting the eggs produced by said poultry and taking therefrom the yolk, for the manufacture of a functional food product, essentially in accordance with what has been described herein before.
Also disclosed herein is the use as defined here above, wherein the functional food product is a product for use in a method of treating and/or preventing a disorder or condition benefiting from supplementation of one or more of omega-3 fatty acids, especially DHA and EPA; vitamin D; Folic acid; Vitamin E; xantophylls, especially lutein and zeaxanthin; iodine; selenium; and zinc. Yet another aspect of the present disclosure concerns a combination of egg yolk solids and dairy solids, especially buttermilk solids. As will be understood by the skilled person, on the basis of the information provided in this application, and in particular from the examples described below, such a combination constitutes a very suitable matrix for the (frequent) oral administration of pharmacologically active substances to a subject, especially fat soluble pharmacologically active substances. Such a matrix affords high biological availability of said pharmacologically active substances, typically owing to certain egg yolk constituents, without the concomitant adverse effects on plasma cholesterol levels normally observed as a consequence of egg yolk consumption.

A particularly suitable matrix in accordance with this aspect of the disclosure comprises at least 10 wt%, on the basis of dry solids weight, of a combination of egg yolk solids and dairy solids, more preferably 15-99.9.wt%, most preferably 25-95 wt%. Said combination typically contains the egg yolk solids and the dairy solids in a ratio within the range of 0.1-100, preferably 1-10, most preferably 2.5-10.

As disclosed herein, said egg yolk solids are selected from egg yolk phospholipids. In another aspect the combination comprises whole egg yolk solids. It is furthermore particularly preferred that said dairy solids are selected from buttermilk solids, skimmed milk solids, semi-skimmed milk solids, fermented milk solids, especially yoghurt solids, fractions thereof and mixtures thereof, most preferably from buttermilk solids and fractions thereof. As further disclosed herein, the combination comprises whole buttermilk solids. As also disclosed herein, the combination comprises one or more polar dairy lipids, preferably selected from the group of phospholipids and sphingolipids, most preferably from sphingolipids. Preferably said lipids are present in an amount of, based on the total weight of the product, at least 0.05 wt%, more preferably at least 0.075 wt%.

The present disclosure also concerns the combination defined before, further comprising an effective amount, e.g. 10⁴ cfu/ml or more, preferably 10⁵ cfu/ml or more, most preferably 10⁶ cfu/ml or more, of lactic acid producing bacteria, more preferably of bacteria selecred from Lactobacillus or Lactococcus, more preferably from *L. Lactis, L. Acidophilus, L. Casei, L. Reuteri, L. Gasseri* and *L. Delbrueckii,* most preferably from *L. Acidophilus, L. Delbrueckii* and *L. Casei.*

Preferably said combination comprises at least one pharmacologically active substance, preferably at least one fat soluble (or lipophilic) pharmacologically active substance, typically in an amount of at least 0.01 wt%, more preferably 0.05-10 wt%, most preferably 0.1-5 wt%.

The above-described combinations of egg yolk solids and buttermilk solids are particularly suitable i) for use as a matrix for the oral administration of one or more lipophilic pharmacologically active substance; ii) for increasing the oral bio-availability of one or more lipophilic phramacoligically active substances; iii) for use in a method of treating a subject in need thereof, said method comprising the administration of a lipophilic pharmacologically active substance dispersed in said combination of egg yolk solids and buttermilk solids; and/or iv) for use in the manufacture of a pharmaceutical or nutraceutical formulation for improving the oral bio-availability of lipophilic pharmacologically active substances. These uses as such as well as the above-described combinations presented or packaged for these uses are also disclosed herein.

### Examples

### Example 1: production of egg yolk enriched in lutein, zeaxanthin or omega-3 fatty acids.

Feeds for producing the enriched eggs were formulated and produced within the legal requirements for animal feed. The use of lutein and zeaxanthin is regulated under EU regulation 1831/2003. The dosage of lutein and zeaxanthin in feed did not exceed the legal limit of 80 ppm in animal feed. For omega 3 fatty acids is a component of some ingredients like linseed meal and fish oil. These products are not limited for use as feed ingredient.

Testing showed the following concentrations for eggs produced by poultry fed the lutein or zeaxanthin enriched feed:

| In egg (mg/egg) | Lutein Mg per egg yolk | | Zeaxanthine Mg per egg yolk | |
|---|---|---|---|---|
| | mean | St dev | mean | St dev |
| Control | 0.16778 | 0.008655 | 0.08504 | 0.001692 |
| Lutein | 0.921422 | 0.105767 | 0.137318 | 0.013953 |
| Zeaxanthine | 0.1743 | 0.014454 | 0.487303 | 0.031019 |

The omega-3 concentration in the eggs produced by poultry fed the omega-3 enriched feed is approximately 200 ± 10mg.

### Example 2: production of functional food products

Two types of products have been prepared containing egg yolk as produced in accordance with example 1. The first product was a 100 ml drink which is intended for daily use (a so-called "shot"). The composition of different types of such drinks is given in table 1 below.

**Table 1**

| **No** | **Contents per 100 ml** | **Form** |
|---|---|---|
| 1 | 20 ml egg yolk (a little over 1 complete egg yolk), 80 ml low-fat yoghurt, 6 gram vanilla sugar | drink |
| 2 | 20 ml egg yolk (a little over 1 complete egg yolk), 80 ml | drink |
| | buttermilk, 6 gram vanilla sugar | |
| 3 | 20 ml egg yolk (a little over 1 complete egg yolk), 20 ml raspberry syrup, 60 ml skimmed milk (0% fat) | drink |
| 4 | 20 ml egg yolk (a little over 1 complete egg yolk), 80 ml yogho yogho^{tm} (a fruit (peach) flavoured yoghurt drink) and 2.5 gram vanilla sugar | drink |
| 5 | 20 ml egg yolk (a little over 1 complete egg yolk), 80 ml orange juice | drink |
| 6 | 20 ml egg yolk (a little over 1 complete egg yolk), 80 ml multifruit juice | drink |

These drinks were all suitable for direct consumption (i.e. ready-to-use). The shelf stability was found to be sufficient for this type of product.

The second product prepared in this example was a 150 ml dessert (a pudding) which is intended for daily use. The composition of the dessert is given in table 2 below.

**Table 2:**

| **no** | **Contents** | **form** |
|---|---|---|
| 7 | 1 liter semi-skimmed milk, 1 vanilla pod, 6 complete egg yolks, 40 gram custard, 60 gram sugar | dessert |

This dessert was suitable for direct consumption (i.e. ready-to-use). The shelf stability was sufficient for this type of product.

### Example 3: use of the functional food product

A randomized placebo-controlled trial is set up using eggs and product produced therefrom in accordance with the present disclosure, said eggs being enriched in lutein, zeaxanthin and omega 3 fatty acids.

The group of test-subjects is divided into 5 groups, consuming either no eggs, normal eggs (lutein: 0.168 ± 0.08, zeaxanthin 0.085 ± 0.0017), eggs enriched with lutein (lutein: 0.921 ± 0.106, zeaxanthin: 0.137 ± 0.014), eggs enriched with zeaxanthin (lutein: 0.174 ± 0.014, zeaxanthin: 0.487 ± 0.031), or egg product made from the lutein enriched eggs, respectively. The omega-3 concentration is the same for all eggs and is 200 ± 10mg.

Boiled eggs were prepared and packed in board or plastic containers and distributed to the test subjects. Also a buttermilk drink containing egg yolk of the enriched eggs was prepared and packed in 100 ml bottles and distributed to the test subjects. The butter-milk drink had the same composition as product no. 2 of example 2. The expiration time for eggs is 28 days. By using boiled eggs stored in a refrigerator this shelf live can be extended to 8 weeks. The buttermilk product, which is pasteurized, tested for cooled shelf live for a period of 6 weeks, and 4 weeks at room temperature.

Each test-subject is asked to consume one egg or egg product daily for a period of 90 days. These are either modified or control eggs or a butter-milk drink in accordance with the disclosure or a control butter-milk drink product. The products are consumed at lunch time.

During this period the subjects are asked to fill in questionnaires which are aimed at establishing the willingness of the subject to comply with the treatment regimen, i.e. the willingness to take the egg or the product of the disclosure on a daily basis. It is especially aimed to establish whether subjects developed disinterest or dislike towards the product.

The study includes follow up time after the 3 months actual trial such that the total study time will be 2 years. During the 3 month period there are 3 measuring point at predetermined intervals. At every measuring point (days 1, 45 and 90) the subjects undergo invasive and non-invasive measuring techniques.

For the invasive part of the study blood samples were taken, and the serum levels of cholesterol, lutein, zeaxanthin, triglycerides and lipoproteins were determined. Table 3 shows the descriptives of 5 groups. In total there were 96 subjects which completed the whole study. There were no statistical significant deferences between the groups for both age (p=0.434) and gender (p=0.993).

**Table 3**

| **Diet** | **Age** | **Male** | **Female** |
|---|---|---|---|
| Normal Egg | 52.8±12.0 | 9 | 11 |
| Lutein Beverage | 43.3±16.1 | 8 | 12 |
| Lutein Egg | 44.6±19.1 | 8 | 12 |
| Control | 44.5±15.8 | 9 | 11 |
| Zeaxanthin Egg | 47.8±17.2 | 9 | 11 |
| P-value | 0.343 | 0.993 | |

### Lipids

For the lipids three groups were compared, namely the control group, beverage group , and the eggs-group - normal, lutein, and zeaxanthin eggs - together. An ANOVA analysis revealed no difference in total cholesterol (p=0.951), LDL cholesterol (p=0.969), HDL cholesterol (p=0.877), or triglycerides (p=0.542) at baseline (see Table 2).

### Total Cholesterol

Analysis of the effect of egg consumption on the total cholesterol levels showed no significant change in total cholesterol between baseline and endpoint between the three groups (P=0.163). Stratification for gender did however showed a significant effect in the female population (P=0.018).

### LDL

An ANOVA comparison showed no significant change in LDL between the three groups (P=0.300), stratification for gender showed a significant contribution of eggs in the female population (P=0.025).

### HDL

Analysis of changes in plasma HDL concentration revealed no significant change between the groups (p=0.257).

### Triglycerides

There was no significant difference in triglycerides change in between the three groups (P=0.170).

### Lutein

At baseline there was a statistically significant higher plasma lever of lutein in the lutein egg group as compared to the lutein beverage group (p=0.036) but not with to the control group (p=0.058, table 3). Stratification for gender resulted in no significant difference at baseline. For zeaxanthin there was no difference between the 5 groups There was a significant change in plasma lutein level between baseline and end point for both the lutein beverage as the lutein egg group when compared to the control group (P<0.001 in both case).

A repeated measurement model showed the same results with p<0.001 for lutein beverage and lutein egg group when compared to the control group.

A mixed model analysis of the measurement with subject number as grouping factor, plasma lutein levels as dependant, diets as factors and week as covariate revealed a significant effect of week and lutein beverage and lutein egg group as compared to the control group(p<0.001), the zeaxanthin group did not reach significance (p=0.080).

### Zeaxanthin

At baseline there was no difference in plasma levels of zeaxanthin between the 5 groups (p=0.482).
The change after 90 days was significantly different for the zeaxanthin egg group when compared to the control group (p<0.001).
A repeated measurement model resulted in a significant difference for both the as the zeaxanthin egg when compared to the control group(P<0.001 respectively).
A mixed model analysis of the measurement with subject number as grouping factor, plasma zeaxanthin levels as dependant, diets as factors and week as covariate revealed a significant effect of week and all diets as compared to the control group, the normal egg group (p=0.050), lutein beverage (p=0.042), the lutein egg (p=0.006), and the zeaxanthin egg group (p<0.001).

The complete results of the serum analyses of the subjects of the 5 test groups is given in the following tables 4 and 5.

**Table 4**

| | **Baseline** | | | **Midpoint** | | | **Endpoint** | | | **Change** | | |
|---|---|---|---|---|---|---|---|---|---|---|---|---|
| **Total Cholesterol** | **Both** | **Male** | **Female** | **Both** | **Male** | **Female** | **Both** | **Male** | **Female** | **Both** | **Male** | **Female** |
| **Control** | 5.48±1.06 | 5.22±1.06 | 5.69±1.06 | 5.52±0.93 | 5.23±0.92 | 5.76±0.91 | 5.28±0.87 | 5.26±0.76 | 5.29±0.99 | -0.20±0.76 | 0.04±0.81 | -0.40±0.69 |
| **Lutein Beverage** | 5.46±1.00 | 5.44±1.09 | 5.48±0.99 | 5.46±1.10 | 5.40±1.33 | 5.51±0.97 | 5.44±1.13 | 5.47±1.22 | 5.41±1.12 | -0.03±0.56 | 0.04±0.51 | -0.07±0.60 |
| **Eggs** | 5.54±0.99 | 5.66±0.94 | 5.44±1.03 | 5.60±1.07 | 5.68±0.99 | 5.53±1.14 | 5.66±1.10 | 5.65±1.07 | 5.68±1.14 | 0.13±0.67 | -0.01±0.73 | 0.24±0.62 |
| **P-value** | 0.951 | 0.510 | 0.789 | 0.881 | 0.509 | 0.803 | 0.343 | 0.628 | 0.557 | 0.163 | 0.976 | 0.018 |
| **LDL-Cholesterol** | | | | | | | | | | | | |
| **Control** | 3.59±1.05 | 3.53±0.96 | 3.64±1.17 | 3.56±0.91 | 3.51±0.76 | 3.60±1.05 | 3.42±0.87 | 3.62±0.59 | 3.25±1.05 | -0.17±0.75 | 0.09±0.80 | -0.38±0.67 |
| **Lutein Beverage** | 3.58±0.92 | 3.64±1.15 | 3.54±0.78 | 3.56±1.05 | 3.60±1.39 | 3.53±0.82 | 3.55±1.06 | 3.71±1.18 | 3.44±1.01 | -0.04±0.53 | 0.07±0.47 | -0.11±0.57 |
| **Eggs** | 3.63±0.93 | 3.93±0.91 | 3.40±0.88 | 3.67±0.97 | 3.91±0.87 | 3.48±1.01 | 3.71±1.01 | 3.92±1.00 | 3.56±1.00 | 0.08±0.60 | -0.01±0.70 | 0.15±0.51 |
| **P-value** | 0.969 | 0.510 | 0.744 | 0.855 | 0.494 | 0.942 | 0.492 | 0.700 | 0.692 | 0.300 | 0.902 | 0.025 |
| **HDL Cholesterol** | | | | | | | | | | | | |
| **Control** | 1.67±0.39 | 1.49±0.25 | 1.82±0.43 | 1.75±0.45 | 1.52±0.31 | 1.94±0.47 | 1.65±0.39 | 1.46±0.22 | 1.81±0.44 | -0.02±0.19 | -0.02±0.10 | -0.01±0.24 |
| **Lutein Beverage** | 1.65±0.35 | 1.53±0.35 | 1.73±0.35 | 1.66±0.36 | 1.50±0.39 | 1.77±0.32 | 1.64±0.43 | 1.44±0.41 | 1.77±0.41 | -0.01±0.24 | -0.09±0.27 | 0.04±0.20 |
| **Eggs** | 1.71±0.50 | 1.51±0.35 | 1.85±0.55 | 1.74±0.49 | 1.57±0.38 | 1.88±0.49 | 1.76±0.53 | 1.53±0.38 | 1.95±0.56 | 0.06±0.21 | 0.01±0.17 | 0.10±0.24 |
| **P-value** | 0.877 | 0.957 | 0.770 | 0.769 | 0.905 | 0.650 | 0.496 | 0.801 | 0.509 | 0.257 | 0.448 | 0.366 |
| **Triglycerides** | | | | | | | | | | | | |
| **Control** | 1.10±0.70 | 1.03±0.44 | 1.16±0.88 | 1.07±0.59 | 1.03±0.48 | 1.10±0.69 | 1.02±0.57 | 0.86±0.33 | 1.16±0.69 | -0.08±0.29 | -0.18±0.23 | 0.00±0.32 |
| **Lutein Beverage** | 1.16±0.79 | 1.34±1.12 | 1.05±0.50 | 1.23±0.81 | 1.50±1.20 | 1.05±0.37 | 1.26±0.96 | 1.59±1.39 | 1.04±0.49 | 0.09±0.39 | 0.25±0.51 | -0.01±0.27 |
| **Eggs** | 0.98±0.61 | 1.05±0.64 | 0.93±0.64 | 0.91±0.56 | 1.02±0.68 | 0.82±0.45 | 0.92±0.62 | 1.01±0.70 | 0.85±0.55 | -0.06±0.34 | -0.04±0.38 | -0.08±0.31 |
| **P-value** | 0.542 | 0.593 | 0.573 | 0.138 | 0.293 | 0.176 | 0.181 | 0.153 | 0.271 | 0.170 | 0.075 | 0.694 |

**Table 5**

| **Lutein** | **Baseline** | **SD-0** | **Midpoint** | **SD-6** | **Endpoint** | **SD-12** | **Change** | **Sd-Change** | **Significance** |
|---|---|---|---|---|---|---|---|---|---|
| Normal Egg | 216,31 | 105,82 | 238,69 | 111,13 | 234,91 | 92,90 | 20,616 | 42,46 | |
| Lutein Beverage | 173,82 | 69,25 | 284,06 | 125,65 | 310,19 | 134,99 | 136,370 | 72,96 | 0,00 |
| Lutein Egg | 289,64 | 212,66 | 437,78 | 195,53 | 464,82 | 200,76 | 175,177 | 112,98 | 0,00 |
| Control | 180,11 | 51,19 | 188,20 | 71,52 | 177,32 | 54,72 | -2,799 | 30,47 | |
| Zeaxanthin Egg | 217,31 | 104,24 | 232,26 | 124,70 | 248,13 | 118,10 | 30,811 | 47,79 | |
| Significance | 0,030 | | 0,000 | | 0,000 | | 0,000 | | |

| **Zeaxanthin** | **Baseline** | **SD-0** | **Midpoint** | **SD-6** | **Endpoint** | **SD-12** | **Change** | **Sd**-**Change** | **Significance** |
|---|---|---|---|---|---|---|---|---|---|
| Normal Egg | 27,81 | 19,28 | 40,87 | 23,41 | 45,62 | 25,29 | 15,842 | 3,63 | |
| Lutein Beverage | 23,55 | 14,51 | 37,83 | 19,78 | 45,39 | 18,14 | 14,723 | 3,29 | |
| Lutein Egg | 27,50 | 16,42 | 45,87 | 16,16 | 52,83 | 23,29 | 19,767 | 4,53 | |
| Control | 20,41 | 10,24 | 28,81 | 22,42 | 26,77 | 10,63 | 7,769 | 1,74 | |
| Zeaxanthin Egg | 29,23 | 23,23 | 127,75 | 65,03 | 127,45 | 54,30 | 42,271 | 9,70 | 0,00 |
| Significance | 0,482 | | 0,000 | | 0,000 | | 0,000 | | |

For the non-invasive part of the study the subjects underwent the following tests: mean visual acuity test using the Early Treatment Diabetic Retinopathy Study (ETDRS)-chart, contrast sensitivity using the Pelli-Robson chart, Scanning Laser Ophthalmoscope (SLO), Optical Coherence Tomography (OCT), MPOD using Heterochromatic Flicker Photometry (HFP), Macular Photocoagulation Study (MPS) and macular pigment Reflectometry (MPR).

### Macular pigment

At baseline there were no significant difference between the 5 groups with any of the measuring devices (p>0.356).

However, a repeated measurement analysis shows a significant contribution of both the normal egg as the lutein egg diet for the MPR-lutein measurement in the female population (p=0.025 and p=0.041 respectively).

### MPS

A mixed model analysis of the measurement with subject number as grouping factor, MPS as dependant, diets as factors and week as covariate showed no significant effect of week and diets as compared to the control group (p>0.237).

### MPR Lutein

At mid point there was a significant difference between the lutein egg group and control group for the MPR-lutein measurement (p=0.046) but this disappeared by end point. Stratification for gender showed the difference at midpoint for the MPR-lutein to be in the female population (p=0.023).

A mixed model analysis of the measurement with subject number as grouping factor, MPR lutein-MPOD as dependant, diets as factors and week as covariate revealed a significant effect of week and lutein egg group as compared to the control group(p=0.006). Stratification for gender showed a significant effect of week and lutein beverage (p=0.039) on lutein fraction of the MPOD compared to the control group in the male population. The lutein egg group did not reach significance (p=0.109). In the female population there were statically significant changes in both the lutein beverage and lutein egg group as compared to the control group (p=0.024 and p=0.016 respectively).

### MPR Zeaxanthin

A mixed model analysis of the measurement with subject number as grouping factor, MPR zeaxanthin-MPOD as dependant, diets as factors and week as covariate revealed no significant effect of week and diets as compared to the control group(p>0.291). Stratification for gender showed a significant effect of week and lutein beverage (p=0.007) on zeaxanthin fraction of MPOD compared to the control group in the male population. The normal egg group and lutein egg group both reached significance (p=0.031 and p=0.026 respectively) in the female population.

### MPR MPOD

A mixed model analysis of the measurement with subject number as grouping factor, MPR-MPOD as dependant, diets as factors and week as covariate revealed no significant effect of week and diets as compared to the control group(p=0.091). Stratification for gender showed a significant effect of week and lutein beverage (p=0.003) on MPOD as compared to the control group in the male population. In the female population there were also significant changes between the normal egg group and lutein egg group as compared to the control group(p=0.032 and p=0.015 respectively).

### SLO

A mixed model analysis of the measurement with subject number as grouping factor, SLO as dependant, diets as factors and week as covariate revealed a significant effect of week and zeaxanthin group compared to the control group(p=0.009), the lutein group did not reach significance (p=0.057). Stratification for gender showed a significant effect of week and lutein beverage(p=0.013), lutein egg(p=0.036), and zeaxanthin(p=0.007) egg on MPOD compared to the control group in the female population. There were no significant changes in the male population.

Comprehensive results of the non-invasive analyses of the subjects of the 5 test groups is given in the following table 6.

**Table 6**

| **MPS** | **Baseline** | **SD**-**0** | **Midpoint** | **SD-6** | **Endpoint** | **SD-12** | **Change** | **Sd**-**Change** |
|---|---|---|---|---|---|---|---|---|
| Normal Egg | 0,35 | 0,18 | 0,31 | 0,10 | 0,32 | 0,16 | -0,001 | 0,12 |
| Lutein Beverage | 0,32 | 0,15 | 0,33 | 0,14 | 0,32 | 0,16 | 0,006 | 0,12 |
| Lutein Egg | 0,42 | 0,23 | 0,34 | 0,10 | 0,38 | 0,14 | -0,048 | 0,20 |
| Control | 0,36 | 0,23 | 0,33 | 0,16 | 0,35 | 0,17 | -0,013 | 0,22 |
| Zeaxanthin Egg | 0,31 | 0,16 | 0,32 | 0,15 | 0,34 | 0,13 | 0,012 | 0,13 |
| Significance | 0,378 | | 0,985 | | 0,788 | | 0,811 | |

| **MPR** | **Baseline** | **SD**-**0** | **Midpoint** | **SD-6** | **Endpoint** | **SD-12** | **Change** | **Sd**-**Change** |
|---|---|---|---|---|---|---|---|---|
| Normal Egg | 0,61 | 0,14 | 0,60 | 0,15 | 0,20 | 0,05 | -0,078 | 0,23 |
| Lutein Beverage | 0,59 | 0,22 | 0,54 | 0,24 | 0,25 | 0,06 | -0,103 | 0,27 |
| Lutein Egg | 0,65 | 0,16 | 0,67 | 0,16 | 0,19 | 0,04 | -0,010 | 0,08 |
| Control | 0,62 | 0,22 | 0,56 | 0,26 | 0,25 | 0,06 | -0,078 | 0,24 |
| Zeaxanthin Egg | 0,65 | 0,14 | 0,59 | 0,19 | 0,24 | 0,06 | -0,046 | 0,24 |
| Significance | 0,752 | | 0,395 | | 0,181 | | 0,751 | |

| **MPR-Lutein** | **Baseline** | **SD**-**0** | **Midpoint** | **SD-6** | **Endpoint** | **SD-12** | **Change** | **Sd**-**Change** |
|---|---|---|---|---|---|---|---|---|
| Normal Egg | 0,17 | 0,06 | 0,18 | 0,05 | 0,18 | 0,06 | 0,006 | 0,08 |
| Lutein Beverage | 0,16 | 0,06 | 0,15 | 0,06 | 0,15 | 0,07 | -0,003 | 0,07 |
| Lutein Egg | 0,19 | 0,07 | 0,21 | 0,05 | 0,21 | 0,06 | 0,015 | 0,07 |
| Control | 0,18 | 0,04 | 0,16 | 0,06 | 0,16 | 0,06 | -0,021 | 0,06 |
| Zeaxanthin Egg | 0,19 | 0,04 | 0,19 | 0,07 | 0,16 | 0,08 | -0,026 | 0,06 |
| Significance | 0,356 | | 0,011 | | 0,147 | | 0,279 | |

| **MPR-** | **Baseline** | **SD**-**0** | **Midpoint** | **SD-6** | **Endpoint** | **SD-12** | **Change** | **Sd**-**Change** |
|---|---|---|---|---|---|---|---|---|
| Normal Egg | 0,44 | 0,13 | 0,43 | 0,13 | 0,38 | 0,17 | -0,084 | 0,21 |
| Lutein Beverage | 0,42 | 0,18 | 0,39 | 0,20 | 0,32 | 0,18 | -0,090 | 0,22 |
| Lutein Egg | 0,46 | 0,15 | 0,45 | 0,13 | 0,45 | 0,14 | -0,025 | 0,09 |
| Control | 0,44 | 0,18 | 0,40 | 0,22 | 0,39 | 0,20 | -0,057 | 0,18 |
| Zeaxanthin Egg | 0,47 | 0,14 | 0,40 | 0,14 | 0,45 | 0,22 | -0,021 | 0,22 |
| Significance | 0,911 | | 0,771 | | 0,237 | | 0,719 | |

| **SLO** | **Baseline** | **SD**-**0** | **Midpoint** | **SD-6** | **Endpoint** | **SD-12** | **Change** | **Sd**-**Change** |
|---|---|---|---|---|---|---|---|---|
| Normal Egg | 0,32 | 0,08 | 0,31 | 0,07 | 0,31 | 0,07 | -0,009 | 0,07 |
| Lutein Beverage | 0,32 | 0,08 | 0,31 | 0,07 | 0,32 | 0,10 | 0,000 | 0,07 |
| Lutein Egg | 0,31 | 0,07 | 0,33 | 0,09 | 0,31 | 0,06 | 0,005 | 0,06 |
| Control | 0,31 | 0,06 | 0,30 | 0,09 | 0,27 | 0,07 | -0,042 | 0,06 |
| Zeaxanthin Egg | 0,32 | 0,06 | 0,32 | 0,07 | 0,34 | 0,09 | 0,003 | 0,07 |
| Significance | 0,977 | | 0,643 | | 0,183 | | 0,372 | |

The results of the first 3 month trial period show that the product as disclosed herein is a suitable vehicle for delivering lutein and zeaxanthin, as evidenced by the results of the analyses of the blood samples. Furthermore, it was established that the level of macular pigment increased throughout the study. The current belief is that lutein and zeaxanthin accumulated in the macular region can help in the prevention of AMD by absorbing this blue light and protecting the retina from oxidative stress by neutralizing free radicals. The possible protective nature of omega-3 is believed to work by regulating inflammatory and immune response in the retina, repairing damaged cells, and improving endothelial cell function. Furthermore, the clinical trial results suggest that compliance by subjects taking the product as disclosed herein will be significantly higher then that by subjects taking eggs, when the regimen comprises daily intake for periods of several months to even years. Finally, the clinical trial results show that the daily intake of an egg yolk product containing buttermilk did not give rise to significant adverse changes in plasma cholesterol levels, triglyceride levels and lipoproteins.

## Claims

1. Method for increasing the efficiency of the absorption of xanthophylls in the gastro-intestinal tract wherein a food product is administered to a human or an animal and wherein the food product comprises a mixture of an aqueous dispersion and said xanthophylls contained in egg yolk, wherein the egg yolk and the aqueous dispersion are present in the food product in a ratio between 1:2 respectively and 1:7 respectively, or a dried equivalent thereof.

2. Method according to claim 1 wherein the xanthophyll is zeaxantin or lutein.

3. Method according to claims 1 or 2 wherein the food product is a beverage or a dessert.

4. Method according to claim 3 wherein the beverage is a flavoured drink or dairy type drink.

5. Method according to claim 3 wherein the dessert is a pudding, custard or yoghurt.

6. Method according to claims 1 - 5 wherein the food product is provided in unit dosage form.

7. Method according to claims 1 - 6 wherein the aqueous dispersion is selected from the group consisting of skimmed milk, semi-skimmed milk, buttermilk fermented milk, such as yoghurt, soy drink, soy milk, fermented soy milk, fruit juices, fruit purees, syrups, vegetable juices and vegetable purees.

8. Method according to claims 1 - 7 wherein the aqueous dispersion is buttermilk or a buttermilk fraction.

9. Composition comprising a mixture of an aqueous dispersion and xanthophylls contained in egg yolk, wherein the egg yolk and the aqueous dispersion are present in a ratio between 1:2 respectively and 1:7 respectively, or the dried equivalent thereof, for use as a medicament.

10. Composition for use according to claims 9 wherein the aqueous dispersion is selected from the group consisting of skimmed milk, semi-skimmed milk, buttermilk fermented milk, such as yoghurt, soy drink, soy milk, fermented soy milk, fruit juices, fruit purees, syrups, vegetable juices and vegetable purees.

## Patentansprüche

1. Verfahren zum Erhöhen der Wirksamkeit der Absorption von Xanthophyllen in dem Magen-Darm-Trakt, wobei ein Lebensmittelprodukt an einen Menschen oder ein Tier verabreicht wird und wobei das Lebensmittelprodukt ein Gemisch aus einer wässrigen Dispersion und den Xanthophyllen, die in Eigelb enthalten sind, wobei das Eigelb und die wässrige Dispersion in dem Lebensmittelprodukt in einem Verhältnis zwischen 1:2 und 1:7 enthalten sind, oder ein getrocknetes Äquivalent davon umfasst.

2. Verfahren gemäß Anspruch 1, wobei das Xanthophyll Zeaxantin oder Lutein ist.

3. Verfahren gemäß Ansprüchen 1 oder 2, wobei das Lebensmittelprodukt ein Getränk oder ein Dessert ist.

4. Verfahren gemäß Anspruch 3, wobei das Getränk ein aromatisiertes Getränk oder ein Getränk vom Milchprodukt-Typ ist.

5. Verfahren gemäß Anspruch 3, wobei das Dessert ein Pudding, eine Creme oder ein Joghurt ist.

6. Verfahren gemäß Ansprüchen 1-5, wobei das Lebensmittelprodukt in Einheitsdosierungsform bereitgestellt ist.

7. Verfahren gemäß Ansprüchen 1-6, wobei die wässrige Dispersion ausgewählt ist aus der Gruppe bestehend aus Magermilch, halbfetter Milch, Buttermilch, fermentierter Milch, wie z. B. Joghurt, Sojagetränk, Sojamilch, fermentierter Sojamilch, Fruchtsäften, Fruchtpürees, Sirupen, Gemüsesäften und Gemüsepürees.

8. Verfahren gemäß Ansprüchen 1-7, wobei die wässrige Dispersion Buttermilch oder eine Buttermilchfraktion ist.

9. Zusammensetzung, umfassend ein Gemisch aus einer wässrigen Dispersion und Xanthophyllen, die in Eigelb enthalten sind, wobei das Eigelb und die wässrige Dispersion in einem Verhältnis zwischen 1:2 und 1:7 vorhanden sind, oder das getrocknete Äquivalent davon, für die Verwendung als Medikament.

10. Zusammensetzung für die Verwendung gemäß Anspruch 9, wobei die wässrige Dispersion ausgewählt ist aus der Gruppe bestehend aus Magermilch, halbfetter Milch, Buttermilch, fermentierter Milch, wie z. B. Joghurt, Sojagetränk, Sojamilch, fermentierter Sojamilch, Fruchtsäften, Fruchtpürees, Sirupen, Gemüsesäften und Gemüsepürees.

## Revendications

1. Méthode d'augmentation de l'efficacité d'absorption de xanthophylles dans le tractus gastro-intestinal où un produit alimentaire est administré à un être humain ou un animal et où le produit alimentaire comprend un mélange d'une dispersion aqueuse et lesdites xanthophylles contenues dans du jaune d'oeuf, où le jaune d'oeuf et la dispersion aqueuse sont présents dans le produit alimentaire selon un rapport compris entre 1:2 respectivement et 1:7 respectivement, ou un équivalent séché de ceux-ci.

2. Méthode selon la revendication 1, dans laquelle la xanthophylle est la zéaxanthine ou la lutéine.

3. Méthode selon les revendications 1 ou 2, dans laquelle le produit alimentaire est une boisson ou un dessert.

4. Méthode selon la revendication 3, dans laquelle la boisson est une boisson aromatisée ou une boisson de type laitier.

5. Méthode selon la revendication 3, dans laquelle le dessert est une crèmedessert, une crème pâtissière ou un yaourt.

6. Méthode selon les revendications 1-5, dans laquelle le produit alimentaire est fourni sous forme de dosage unitaire.

7. Méthode selon les revendications 1-6, dans laquelle la dispersion aqueuse est choisie dans le groupe constitué par le lait écrémé, le lait demi-écrémé, le babeurre, le lait fermenté, tel que le yaourt, une boisson au soja, le lait de soja, le lait de soja fermenté, les jus de fruits, les purées de fruits, les sirops, les jus de légumes et les purées de légumes.

8. Méthode selon les revendications 1-7, dans laquelle la dispersion aqueuse est le babeurre ou une fraction de babeurre.

9. Composition comprenant un mélange d'une dispersion aqueuse et de xanthophylles contenues dans du jaune d'oeuf, où le jaune d'oeuf et la dispersion aqueuse sont présents selon un rapport compris entre 1:2 respectivement et 1:7 respectivement, ou l'équivalent séché de ceux-ci, pour une utilisation comme médicament.

10. Composition pour une utilisation selon la revendication 9, dans laquelle la dispersion aqueuse est choisie dans le groupe constitué par le lait écrémé, le lait demi-écrémé, le babeurre, le lait fermenté, tel que le yaourt, une boisson au soja, le lait de soja, le lait de soja fermenté, les jus de fruits, les purées de fruits, les sirops, les jus de légumes et les purées de légumes.
